(19) Europäisches Patentamt
European Patent Office
Office européen des brevets

(11) Veröffentlichungsnummer: **0 005 276**
**B1**

(12) # EUROPÄISCHE PATENTSCHRIFT

(45) Veröffentlichungstag der Patentschrift: **07.01.81**

(51) Int. Cl.³: **C 07 C 157/09**

(21) Anmeldenummer: **79101402.0**

(22) Anmeldetag: **08.05.79**

(54) **Verfahren zur Herstellung von Monoarylthioharnstoffen.**

(30) Priorität: **10.05.78 DE 2820321**

(43) Veröffentlichungstag der Anmeldung: **14.11.79 Patentblatt 79/23**

(45) Bekanntmachung des Hinweises auf die Patenterteilung: **07.01.81 Patentblatt 81/1**

(84) Benannte Vertragsstaaten: **BE CH DE FR GB IT**

(56) Entgegenhaltungen: **DE-C-604 639**

(73) Patentinhaber: **HOECHST AKTIENGESELLSCHAFT, Zentrale Patentabteilung Postfach 80 03 20, D-6230 Frankfurt/Main 80 (DE)**

(72) Erfinder: **Papenfuhs, Theodor, Dr., Heinrich-Bleicher-Strasse 40, D-6000 Frankfurt/Main 50 (DE)**

EP 0 005 276 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

ACTORUM AG.

# Verfahren zur Herstellung von Monoarylthioharnstoffen

Die Erfindung liegt in der Verfahrensverbesserung zur Herstellung von Monoarylthioharnstoffen, deren technische Verwendung aus Houben-Weyl, Methoden der organischen Chemie, 4. Auflage, Band 9, S. 884 (1955), und Chem. Rev. 55, 181 (1955) bekannt ist; sie können auch als Vorprodukte für die Herstellung von Azofarbstoffen dienen.

Von den literaturbekannten Wegen zur Herstellung von Arylthioharnstoffen (s. Houben-Weyl, loc. cit., S. 885ff.) wird technisch die Umsetzung von Arylaminen mit Rhodaniden genutzt. Die Reaktion ist prinzipiell im wässrigen Medium durchführbar, wobei die Arylamine von Vorteil als mineralsaure Salze eingesetzt werden. Es entstehen aber neben den gewünschten Monoarylthioharnstoffen in unterschiedlicher Menge auch N,N′-Diarylthioharnstoffe, deren Menge nach Houben-Weyl (loc. cit. S. 887) mit steigender Konzentration der Arylaminsalze zunimmt (vgl. auch A. Bertram, Ber. Dtsch. Chem. Ges. 25, 48 (1892)). Die Reaktion im organischen Lösungsmittel (bspw. Chloraromaten, Chloraliphaten, Alkohole) verläuft einheitlicher (s. Org. Synth., Coll. Vol. III, S. 77; J. Journ. Med. Chem. 15 (1972), 1024; Japanische Patentbekanntmachung Sho-49-1255); sie ist daher technisch bevorzugt.

Trotzdem hat es nicht an Versuchen gefehlt, die Synthese von Arylthioharnstoffen in wässrigem Medium hinsichtlich Ausbeute und Entstehung einheitlicher Produkte so weit zu verbessern, dass sie auch in wirtschaftlicher Sicht mit den Lösungsmittelverfahren, die in sehr guter Ausbeute verlaufen, konkurrenzfähig ist. Um die Diarylthioharnstoff-Bildung zu unterdrücken, wurde ausnahmslos versucht, in höherer Verdünnung zu arbeiten (vgl. Chem. Ind. (London) 1974, Seiten 18 und 750; belgische Patentschrift 603595; belgische Patentschrift 765477; Helv. Chim. Acta 25, 515 (1942); Org. Synth., Coll. Vol. IV, S. 180). Häufig wurde zur Erzielung zufriedenstellender Ausbeuten ein grosser Überschuss an dem Rhodanid als zweite Ausgangsverbindung und an Mineralsäure benötigt. Die Aufarbeitungsmethoden dieser bekannten Verfahren sind meist sehr aufwendig, beispielsweise wegen mehrmaliger Eindampfoperationen zur Trockne, die Reaktionszeiten sehr lang, die Raum/Zeit-Ausbeuten zu niedrig, die Verfahren somit insgesamt technisch unbefriedigend. Die Ausbeuten an dem Arylthioharnstoff, die nach diesen bekannten Verfahren erzielbar sind, liegen unter 75% der Theorie, in der Regel nur zwischen 30 und 60% der Theorie; die Verfahren sind damit wirtschaftlich uninteressant, zumal sie zusätzlich eine hohe Abwasserbelastung mit sich bringen, insbesondere dann, wenn ein Überschuss an dem Rhodanid und an Säure verwendet wird.

Der Zweck der bekannten Verfahren, in hohen Verdünnungen zu arbeiten (die Ausgangskonzentrationen an den als Ausgangsverbindungen eingesetzten Arylaminsalzen liegen zwischen 4 Gew.-% und maximal 35 Gew.-%), war, die Bildung der Diarylthioharnstoffe weitgehend zu unterdrücken. Trotz dieser Massnahme und zusätzlich zu den oben genannten Nachteilen ist es bei diesem bekannten Verfahren meist unumgänglich, eine Reinigung der erhaltenen Rohprodukte vorzunehmen.

Wegen dieser schwerwiegenden Nachteile haben alle literaturbekannten Verfahren der wässrigen Umsetzung von Arylaminen mit Rhodaniden bisher keine technische Bedeutung erlangt.

Es wurde nun ein Verfahren gefunden, das zu einheitlichen Monoarylthioharnstoffen führt, bei welchem man Salze aromatischer Amine mit Ammoniumrhodaniden oder Alkalirhodaniden in wässrigem Medium umsetzt. Die Verfahrensverbesserung wird dadurch bewirkt, dass man von einem wässrigen Reaktionsgemisch ausgeht, in welchem die Menge an dem Salz des aromatischen Amins in einer grösseren Menge als das Wasser, das als Reaktionsmedium dient, enthalten ist und in welches das Ammonium- oder Alkalirhodanid in mindestens der stöchiometrisch erforderlichen Menge und höchstens mit einem 25%igen molaren Überschuss eingesetzt wird und welches keine überschüssige Säure besitzt, und dass man die Reaktion bei einer Temperatur ausführt, die mindestens 5 °C, vorzugsweise mindestens 10 °C, unter dem Siedepunkt des Reaktionsgemisches liegt, wobei der Siedepunkt des Reaktionsgemisches im wesentlichen abhängig von dem angewandten Druck (Normaldruck oder erhöhter Druck im geschlossenen Gefäss oder Autoklaven) ist.

Die vorliegende Erfindung betrifft demnach ein Verfahren zur Herstellung von Monoarylthioharnstoff-Verbindungen durch Umsetzung von einem Salz eines aromatischen Amins (Arylamins) mit Ammonium- oder einem Alkalirhodanid, wie Natrium- oder Kaliumrhodanid, in wässrigem Medium, das dadurch gekennzeichnet ist, dass man die Reaktion in einem Reaktionsgemisch ausführt, das ausgangs eine höhere Gewichtsmenge an dem Salz des Arylamins als an Wasser und die 1- bis 1,25-fach äquivalente, vorzugsweise 1,05–1,10-fach äquivalente Menge an dem Ammoniumrhodanid oder Alkalirhodanid, bezogen auf das eingesetzte Arylamin, enthält, sowie keine überschüssige Säure aufweist, und die Reaktion bei einer Temperatur ausführt, die mindestens 5 °C, vorzugsweise mindestens 10 °C, unterhalb des Siedepunktes des Reaktionsgemisches liegt.

Das Vorhandensein von freier Säure, die, wie später noch ausgeführt, wegen der Rhodanidzersetzung in gewissem Umfang die Reaktion ungünstig beeinflussen kann, ist nicht zu vermeiden, da wegen der schwachen Basizität der Arylamine zumindestens die Salze der Mineralsäuren mit den Arylaminen in einem chemischen Gleichgewicht mit der dissoziierten Säure vorliegen und somit freie Säure im Reaktionsgemisch vorliegt. Das Arylaminsalz darf deshalb nur in stöchiometrischer Menge mit dessen Säure gebildet werden, und ein Überschuss an Säure ist auszuschliessen.

Die Reaktion des hochkonzentrierten Reaktionsgemisches, das als Suspension vorliegen kann, wird vorteilhaft unter Rühren oder unter ständigem Durchmischen des Reaktionsgemisches durch andere in der Technik übliche Mittel ausgeführt. Um eine ausreichend hohe Reaktionsgeschwindigkeit zu erreichen, wird die Reaktion bevorzugt bei einer Temperatur von mindestens 50°C durchgeführt. Gemäss den bekannten physikalischen Gesetzen ist der Siedepunkt des Reaktionsgemisches abhängig von der Konzentration des wässrigen Reaktionsmediums und dem angewandten Druck. Bei den hier vorliegenden hochkonzentrierten Lösungen bzw. Suspensionen ist bei Normaldruck (atmosphärischer Druck) mit einem Siedepunkt von 107 bis 110°C zu rechnen; bei Anwendung von einem Überdruck von beispielsweise 3 bar, beispielsweise in einem verschlossenen Kessel, liegt der Siedepunkt des Reaktionsgemisches bei etwa 130–135°C. Der genaue Siedepunkt lässt sich für den jeweiligen Fall sehr leicht durch einen Vorversuch ermitteln.

Nach dem erfindungsgemässen Verfahren können leicht und in hoher Ausbeute sowie guter technischer Reinheit Monoarylthioharnstoff-Verbindungen hergestellt werden, deren Schmelzpunkt über 100°C liegt und die im Arylrest unsubstituiert sind oder schwach elektronegative, elektroneutrale und/oder elektropositive Substituenten enthalten. Nitrogruppen, Sulfogruppen und Cyangruppen beispielsweise sind als stark elektronegative Substituenten ausgeschlossen. Der Arylrest im Arylamin als Ausgangsverbindung bzw. im Monoarylthioharnstoff als Endprodukt stellt einen aromatischen carbocyclischen Rest dar, insbesondere einen Benzolkern oder einen Naphthalinkern, die durch schwach elektronegative, elektroneutrale und/oder elektropositive Substituenten substituiert sein können.

Solche Substituenten sind beispielsweise niedere Alkylgruppen, wie Methyl-, Äthyl- oder Propylgruppen, die Hydroxygruppe, niedere Alkoxygruppen, wie Methoxy-, Äthoxy-, Propoxy- oder Butoxygruppen, niedere Alkoxyalkylgruppen, Aryloxygruppen, wie die Phenoxygruppe oder deren durch niederes Alkyl, niederes Alkoxy und/oder Chlor substituierter Rest, ggf. durch niedere aliphatische Reste und/oder Arylreste, wie Phenylreste, substituierte Aminogruppen, Acylaminogruppen oder niedere Alkylestergruppen, wie beispielsweise von niederen aliphatischen Carbonsäuren oder von Arylcarbonsäuren, wie der Benzoesäure, niedere Alkylmercapto- oder Arylthioreste, wie der Phenylthiorest, und Acyloxygruppen von niederen aliphatischen Carbonsäuren oder Arylcarbonsäuren, wie der Benzoesäure. Schwach negative Gruppen sind insbesondere Halogenatome, wie Fluor-, Chlor- oder Bromatome, niedere Alkanoyl- und niedere Alkoxycarbonylgruppen, Aryloylreste, wie der Benzoylrest, die Carbonamid- und die Sulfonamidgruppe, die durch niederes Alkyl und/oder Phenyl mono- oder disubstituiert sein können.

Die hier im folgenden zu findende Angabe «niedere» bezieht sich auf Alkyl- oder Alkylengruppen von 1–4 C-Atomen, die in den erwähnten Verbindungen oder Resten enthalten sein können oder daraus bestehen.

Arylamine, die zwei primäre Aminogruppen enthalten, können je nach Anwendung von einem oder zwei Mol des Rhodanids und der zur Salzbildung erforderlichen Säure gezielt zur Herstellung einer Monoarylthioharnstoff-Verbindung bzw. einer Arylen-bis-thioharnstoff-Verbindung eingesetzt werden.

Das erfindungsgemässe Verfahren verläuft einheitlich und ergibt hohe Ausbeuten an technisch reinen Monoarylthioharnstoffen. Es ist besonders zur Herstellung von Monophenylthioharnstoff- und Mononaphthylthioharnstoff-Verbindungen sowie deren im Benzol- oder Naphthalinkern durch elektropositive und/oder durch schwach elektronegative Gruppen substituierte Derivate geeignet. Zur Herstellung dieser Verbindungen geht man entsprechend von den unsubstituierten bzw. von den durch die obengenannten Gruppen substituierten Phenylaminen bzw. Naphthylaminen aus.

Bevorzugt ist nach dem erfindungsgemässen Verfahren die Herstellung von Monophenyl-thioharnstoff-Verbindungen der allgemeinen Formel (1)

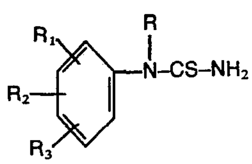

(1)

in welcher R für ein Wasserstoffatom oder eine niedere Alkylgruppe, wie Methyl- oder Äthylgruppe, $R_1$ für ein Wasserstoffatom oder Fluor-, Chlor- oder Bromatom oder eine niedere Alkylgruppe, wie Methyl- oder Äthylgruppe, eine niedere Alkoxygruppe, wie Methoxy- oder Äthoxygruppe, eine Hydroxygruppe, eine Amino-, Phenylamino-, Phenoxy-, niedere Monoalkylamino-, niedere Dialkylamino- oder für die Carbonamidgruppe steht, $R_2$ ein Wasserstoffatom oder Chloratom oder eine niedere Alkylgruppe oder niedere Alkoxygruppe bedeutet sowie $R_3$ ein Wasserstoffatom oder eine niedere Alkylgruppe oder niedere Alkoxygruppe darstellt, wobei die Formelreste R, $R_1$, $R_2$ und $R_3$ gleich oder verschieden voneinander sein können. Zur Herstellung der Verbindungen der Formel (1) geht man entsprechend von den Anilinverbindungen der allgemeinen Formel (2)

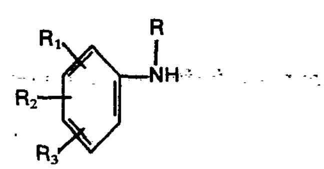

(2)

aus, in welcher R, $R_1$, $R_2$ und $R_3$ die oben genannten Bedeutungen besitzen.

Arylamine, die als Ausgangsverbindungen in das erfindungsgemässe Verfahren mit besonderem Vorteil eingesetzt werden können, sind beispielsweise Anilin, die verschiedenen Toluidine und Xylidine sowie Mesidin, die verschiedenen Anisidine, Phenetidine sowie deren entsprechende Thioätherverbindungen, die verschiedenen Halogenaniline, wobei unter Halogen Fluor, Brom und insbesondere Chlor verstanden wird, die verschiedenen Halogentoluidine und Halogenanisidine, wobei diese Halogenverbindungen das Halogen ein-, zwei- oder dreimal als Substituenten enthalten können, weiterhin die verschiedenen Aminophenole und Phenylendiamine; diese Arylaminoverbindungen können die Aminogruppe in Form des primären Amins als auch in Form eines sekundären Alkylamins besitzen. Sofern das Arylamin lediglich eine primäre Aminogruppe (R=H) aufweist, sind als erfindungsgemäss verwendbare Arylamine beispielsweise die verschiedenen Amino-acetophenone und Aminobenzophenone, des weiteren die Amino-benzoealkylsäureester, die Aminodiphenyläther und -thioäther, die Aminonaphthaline und deren im aromatischen Kern durch niederes Alkyl, niederes Alkoxy und/oder Halogen substituierten Verbindungen zu nennen.

Es ist weiterhin vorteilhaft, jedoch nicht zwingend, die Umsetzung unter Verwendung von 5-10 Mol-% von Hydrogensulfit-Ionen vorzunehmen. Die Gegenwart von Hydrogensulfit-Ionen gewährleistet auch bei technischer Durchführung des Verfahrens eine geruchsneutrale, saubere Abluft. Der empfohlene Zusatz an Hydrogensulfit liegt darin begründet, dass während der erfindungsgemässen Umsetzung eine saure Selbstzersetzung des Rhodanids als nicht vermeidbare Nebenreaktion zu schwefelhaltigen Produkten mit der Oxidationsstufe des Schwefelwasserstoffes auftritt; diese durch die Selbstzersetzung des Rhodanids entstehenden Sulfide werden durch das Hydrogensulfit unter Bildung von Thiosulfat abgefangen. Wegen dieser Nebenreaktion sollte zur Erzielung von hohen Ausbeuten nicht nur die erforderliche stöchiometrische Menge an Rhodanid als zweiter Ausgangskomponente, sondern ein geringer Überschuss in das Reaktionsgemisch eingesetzt werden; aus Gründen der Abwasserbelastung sollte jedoch ein Überschuss an Rhodanid von über 25 Mol-% vermieden werden, obgleich er keinen Einfluss auf die Qualität der Reaktionsprodukte, des Monoarylthioharnstoffs, und auf die Reaktionsführung hat. Aufgrund der sauren Selbstzersetzung des Rhodanids ist deshalb die Gegenwart von freier Säure im Reaktionsgemisch zu vermeiden.

Als Salze von Arylaminen kommen die Arylaminsalze von starken Säuren in Frage, insbesondere die Salze von starken Mineralsäuren, wie Bromwasserstoffsäure, Schwefelsäure, Phosphorsäure und insbesondere Salzsäure. Die Salzbildung aus dem Arylamin und der Säure muss deshalb in stöchiometrisch äquivalenten Mengen erfolgen. Überschuss an Säure führt zu den oben erwähnten Zersetzungsreaktionen des Rhodanids, damit zur Ausbeuteminderung und insbesondere in Folge der entstehenden niedrigwertigen Schwefelverbindungen, die in Form von übelriechenden und giftigen Abgasen frei werden, zu einem technisch nicht statthaften Verfahren. Aber auch bei Vorliegen eines Teils des Arylamins als freie Base durch Säureunterschuss bei der Herstellung bzw. Bildung des Arylaminsalzes ergibt sich eine beträchtliche Ausbeuteminderung, da sich bei Ausführung dieser Verfahrensweise wesentlich unreinere Arylthioharnstoffverbindungen bilden, die nachfolgende Reinigungsoperationen erforderlich machen.

Zur Herstellung des Reaktionsgemisches geht man beispielsweise davon aus, dass man das Arylaminsalz, vorzugsweise das Salz einer Mineralsäure des Arylamins, in einer gewichtsmässig geringeren Menge Wasser löst oder suspendiert, oder dass man das Arylamin in einer stöchiometrischen Menge einer wässrigen Lösung der starken Säure, wie Mineralsäure, löst oder suspendiert, wobei der Wassergehalt der Säurelösung so berechnet ist, dass dessen Gewichtsmenge unter der des Arylaminsalzes bleibt. Zu diesen Arylaminsalzlösungen oder -suspensionen gibt man dann das Rhodanid und gegebenenfalls ein Alkalihydrogensulfit, wie Natriumhydrogensulfit, in fester Form oder in konzentriert wässriger Lösung hinzu, wobei man bei Verwendung wässriger Lösungen beachten muss, dass in dem so hergestellten Reaktionsgemisch die Gesamtmenge an Wasser gewichtsmässig unter der des Arylaminsalzes bleibt.

Das Reaktionsgemisch wird sodann erwärmt, vorzugsweise auf eine Temperatur von über 45°C; die Reaktion kann vorteilhaft sowohl im Temperaturbereich zwischen etwa 50 und 100°C geführt werden ebenso wie in einem Temperaturbereich unterhalb von 5-10°C der Siedetemperatur des Reaktionsgemisches. Dabei sollte die Temperaturführung so ausgewählt werden, dass sie zu optimal reinen Endprodukten mit guten Ausbeuten führt, ohne dass qualitätsmindernde Verunreinigungen im Endprodukt vorhanden sind. Diese Temperaturführung lässt sich für jedes gewünschte Endprodukt in einem Vorversuch leicht ermitteln. Verunreinigungen im Endprodukt und geringere Ausbeuten können nämlich dann entstehen, wenn während der Reaktion des Arylaminsalzes mit dem Rhodanid eine ölige oder schmierige Phase durchlaufen wird, in welcher Ausgangsverbindungen eingeschlossen werden können; hierdurch wird die Reaktion der Komponenten selbst behindert, die Ausbeute somit vermindert und der gewünschte Monoarylharnstoff verunreinigt. Die Gefahr der Verunreinigung ist nicht mehr gegeben, wenn sich das Endprodukt kristallin abzuscheiden beginnt. Die Temperaturführung sollte deshalb so gewählt werden, dass solche öligen oder schmierigen Phasen während des Reaktionsablaufes nicht entstehen; dies kann leicht für jedes Ausgangsgemisch bzw. für jede Reaktion zur Herstellung eines bestimmten gewünschten Endproduktes

durch einen schnellen, für den Fachmann leicht auszuführenden Vorversuch ermittelt werden. Bevorzugte und vorteilhafte Temperaturbereiche der erfindungsgemässen Reaktion für verschiedene Ausgangsamine zur Herstellung der entsprechenden Monoarylthioharnstoff-Endprodukte sind in den nachfolgenden Beispielen angegeben.

Zur Verkürzung der Reaktionszeit kann die Reaktionstemperatur, nachdem die Abscheidung des Endproduktes in kristalliner Form begonnen hat, gesteigert werden, jedoch lediglich auf eine Temperatur, die höchstens 5 °C, vorzugsweise höchstens 10 °C unter dem Siedepunkt des Reaktionsgemisches liegt; zur Anwendung höherer Temperaturen kann unter Druck gearbeitet werden, in der Regel ist jedoch die Durchführung der Reaktion bei Atmosphärendruck völlig ausreichend. Hohe Reaktionstemperaturen von 5 °C unter dem Siedepunkt des Reaktionsgemisches bei eventueller Anwendung von Druck wird man deshalb nur gelegentlich und in Einzelfällen zur Erzielung einer besseren Raum/Zeit-Ausbeute vorziehen.

Die Begrenzung der Reaktionstemperatur, insbesondere der Reaktionsendtemperatur, auf höchstens 5–10 °C unter dem Siedepunkt bestimmt wesentlich die Qualität des Endproduktes. Die erfindungsgemässe Reaktion des Arylaminsalzes mit dem Rhodanid zur gewünschten Monoaryl-thioharnstoff-Verbindung steht nämlich in einem unerwünschten Reaktionsgleichgewicht unter Bildung der entsprechenden Diarylthioharnstoff-Verbindung. Diese Nebenreaktion wird unter den angewandten erfindungsgemässen Bedingungen und Verfahrensparametern, insbesondere durch die Anwendung hoher Konzentrationen an dem Arylaminsalz im Reaktionsgemisch und der Begrenzung der Höchsttemperatur, zurückgedrängt und unterdrückt. Arbeitet man hingegen bei Siedetemperatur selbst, werden die grösstenteils wasserdampfflüchtigen Diarylthioharnstoff-Verbindungen destillativ aus dem Reaktionsgemisch entfernt, indem sie sich im Kühler oder im Destillat abscheiden, wodurch sich das Reaktionsgleichgewicht Ausgangskomponenten/Monarylthioharnstoff/Diarylthioharnstoff laufend neu einstellen muss und aus der bereits entstandenen Monoarylthioharnstoff-Verbindung reversibel Bildung der Diarylthioharnstoff-Verbindung erfolgt. Auf diese Weise verringern sich Ausbeute und Qualität des gewünschten Monoarylthioharnstoffes erheblich.

Insbesondere durch die erfindungsgemäss verwendete hohe Konzentration des Arylaminsalzes in dem Reaktionsgemisch liefert das erfindungsgemässe Verfahren im Vergleich zum Stand der Technik eine erhebliche Steigerung der Ausbeute und ebenso der Reinheit des Produktes; mit dem erfindungsgemässen Verfahren ist eine praktisch quantitative Umsetzung der Ausgangskomponenten zu dem gewünschten Monarylthioharnstoff-Endprodukt innerhalb einer verhältnismässig kurzen und damit wirtschaftlich günstigen Reaktionszeit möglich. Die Ausbeuten liegen im allgemeinen über 90% der Theorie, in der Regel kann mit einer Ausbeute von 95–98% der Theorie gerechnet werden. Die Raum/Zeit-Ausbeute im erfindungsgemässen Verfahren ist sehr hoch, die Abwasserbelastung gegenüber bekannten Verfahren deutlich verringert.

Die nachstehenden Beispiele dienen zur Erläuterung der Erfindung. Die Teile sind Gewichtsteile, die Prozentangaben beziehen sich auf Gewichtsprozent, sofern nichts anderes vermerkt.

Beispiel 1:

In 1050 Teile 20%iger wässriger Salzsäure werden unter Rühren nacheinander 738 Teile p-Anisidin, 502 Teile Ammonium-rhodanid und 78 Teile einer 40%igen wässrigen Natriumbisulfit-Lösung eingetragen. Diese Mischung wird auf 95 bis 100 °C erhitzt und 12 bis 15 Stunden bei dieser Temperatur gerührt. Der gebildete Niederschlag wird danach heiss abgesaugt, mit heissem Wasser neutral gewaschen und getrocknet. Es werden 1038 Teile (entsprechend 95,1% der Theorie) 4-Methoxyphenyl-thioharnstoff der Formel

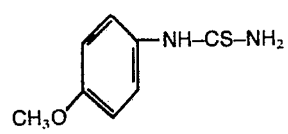

mit einem Schmelzpunkt von 200 bis 201 °C erhalten.

Ersetzt man das Ammoniumrhodanid durch eine äquivalente Menge an Natrium- oder Kaliumrhodanid, so erhält man den 4-Methoxyphenyl-thioharnstoff in gleich guter Ausbeute und Qualität.

Beispiel 2:

In 592 Teilen einer 37%igen wässrigen Salzsäure gibt man nacheinander 558 Teile Anilin, 479 Teile Ammoniumrhodanid und 78 Teile einer 40%igen wässrigen Natriumbisulfitlösung. Dieses Reaktionsgemisch rührt man zunächst acht Stunden bei 80 °C, sodann acht Stunden bei 90 °C, kühlt darauf auf 20 °C ab, saugt den gebildeten Niederschlag ab, wäscht diesen neutral und trocknet ihn. Man erhält 888 Teile (entsprechend 97,4% d.Th.) Phenylthioharnstoff mit einem Schmelzpunkt von 148–150 °C.

Verwendet man anstelle der 37%igen Salzsäure eine äquivalente Menge (= 980 Teile) einer 30%igen wässrigen Schwefelsäure, so erhält man den Phenylthioharnstoff in gleich guter Ausbeute und Qualität.

Beispiel 3:

750 Teile einer 30%igen wässrigen Salzsäure werden in einem Reaktionsgefäss vorgelegt; unter Rühren werden nacheinander 822 Teile p-Phenetidin, 479 Teile Ammoniumrhodanid und 31,2 Teile Natriumhydrogensulfit eingetragen. Das Reaktionsgemisch wird 15 Stunden bei 80 °C, sodann acht Stunden bei 95 °C gerührt; anschliessend

wird der gebildete Niederschlag heiss abgesaugt und mit heissem Wasser neutral gewaschen. Das getrocknete Produkt ergibt eine Ausbeute von 1129 Teile (entsprechend 96,0% d.Th.) p-Äthoxyphenylthioharnstoff der Formel

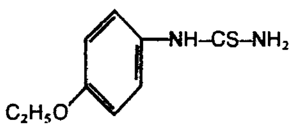

mit einem Schmelzpunkt von 167–168 °C.

Ersetzt man das Natriumbisulfit durch eine äquivalente Menge an Kalium- oder Ammoniumbisulfit, so lässt sich der p-Äthoxyphenyl-thioharnstoff in gleich guter Ausbeute und Qualität herstellen.

Beispiel 4:

Eine Mischung aus 592 Teilen einer 37%igen Salzsäure, 642 Teilen o-Toluidin, 570 Teilen Ammoniumrhodanid und 78 Teilen einer 40%igen wässrigen Natriumbisulfitlösung wird vier Stunden bei 60 °C, sodann noch 4 Stunden bei 70 °C und abschliessend acht Stunden bei 80 °C gerührt, sodann auf 20 °C abgekühlt; der gebildete Niederschlag wird abgesaugt, neutral gewaschen und getrocknet. Man erhält 950 Teile (entsprechend 95,4% d.Th.) 2-Methylphenylthioharnstoff mit einem Schmelzpunkt von 156 °C.

Führt man die Reaktion ohne Natriumbisulfit aus, so erhält man den 2-Methylphenyl-thioharnstoff in gleicher Ausbeute mit wenig verringerter Qualität; er zeigt einen Schmelzpunkt von 154 °C.

Beispiele 5 bis 38:

In gleicher Weise lassen sich die den nachfolgenden Tabellenbeispielen entsprechenden Arylthioharnstoffe der Formel (1) mit den dort angegebenen Ausbeuten und Qualitäten (charakterisiert durch den Schmelzpunkt des erhaltenen Endproduktes) nach den dort angegebenen Reaktionsbedingungen aus den entsprechenden Ausgangs-Arylamin-hydrochloriden (hergestellt aus stöchiometrischen Mengen an dem Arylamin der Formel (2) und wässriger Salzsäure mit dem dort angegebenen Gehalt an HCl) herstellen, wobei diese Arylaminhydrochloride in Form dieser salzsauren Lösung oder Suspension für das Reaktionsgemisch eingesetzt werden.

| Bsp. | Arylamin der Formel (2) (1 Mol) | $NH_4SCN$ (Mol) | %ige HCl | Reaktions- temp. (°C) | Reaktions- zeit (h) | Ausbeute (% d.Th.) | Smp. (°C) |
|---|---|---|---|---|---|---|---|
| 5 | m-Toluidin | 1,05 | 37,0 | 70 | 20 | 86,6 | 110 |
| 6 | p-Toluidin | 1,05 | 20,0 | 80/100 | 2/5 | 91,0 | 188 |
| 7 | o-Anisidin | 1,05 | 20,0 | 80/90 | 8/8 | 95,6 | 152 |
| 8 | m-Anisidin | 1,05 | 20,0 | 80/90 | 8/8 | 90,1 | 160 |
| 9 | m-Aminophenol | 1,05 | 20,0 | 80/90 | 8/8 | 79,0 | >230 |
| 10 | p-Aminophenol | 1,05 | 20,0 | 80/90 | 8/8 | 89,0 | 215 |
| 11 | o-Chloranilin | 1,05 | 20,0 | 80/100 | 8/8 | 98,2 | 146 |
| 12 | m-Chloranilin | 1,05 | 20,0 | 80/90 | 8/12 | 93,3 | 135 |
| 13 | p-Chloranilin | 1,05 | 20,0 | 80/90 | 8/8 | 94,5 | 178 |
| 14 | p-Phenylendiamin | 1,05 | 30,0 | 80/90 | 8/8 | 91,2 | 190 |
| 15 | p-Phenylendiamin | 2,10 | 20,0 | 80/100 | 8/8 | 93,2 | 230 |
| 16 | p-Dimethylamino-anilin | 1,05 | 20,0 | 80/90 | 8/10 | 91,9 | 180 |
| 17 | 2-(β-Methoxy-äthoxy)-anilin | 1,25 | 37,0 | 70 | 20 | 82,8 | 145 |
| 18 | p-Acetylamino-anilin | 1,05 | 20,0 | 80/90 | 8/8 | 88,9 | 201 |
| 19 | p-Aminobenzamid | 1,05 | 20,0 | 80/100 | 4/12 | 87,2 | 229 |
| 20 | p-Aminobenzoesäureäthylester | 1,05 | 20,0 | 80 | 7 | 98,2 | 157 |
| 21 | p-Amino-benzophenon | 1,05 | 20,0 | 80/90 | 8/8 | 89,7 | 179 |
| 22 | 2,4-Dimethyl-anilin | 1,05 | 20,0 | 80/90 | 8/8 | 87,2 | 174 |
| 23 | 2,5-Dimethyl-anilin | 1,05 | 37,0 | 70 | 20 | 95,6 | 136 |
| 24 | 2,4-Dimethoxy-anilin | 1,05 | 20,0 | 70/80 | 5/15 | 81,1 | 194 |
| 25 | 2,5-Dimethoxy-anilin | 1,05 | 20,0 | 70/80 | 5/15 | 91,1 | 163 |
| 26 | 2,5-Diäthoxy-anilin | 1,05 | 20,0 | 70 | 16 | 84,2 | 151 |
| 27 | 2-Methyl-5-chlor-anilin | 1,05 | 30,0 | 80 | 12 | 89,7 | 165 |
| 28 | 2-Methyl-4-chlor-anilin | 1,10 | 20,0 | 80/90 | 4/12 | 93,8 | 153 |
| 29 | 4-Methyl-2-chlor-anilin | 1,05 | 20,0 | 80/90 | 8/8 | 85,9 | 167 |
| 30 | 2-Methoxy-4-chlor-anilin | 1,05 | 20,0 | 70 | 16 | 83,9 | 159 |
| 31 | 2,3-Dichloranilin | 1,05 | 20,0 | 80 | 9 | 86,2 | 140 |
| 32 | 2,4-Dichloranilin | 1,10 | 25,0 | 80/100 | 4/8 | 94,0 | 146 |
| 33 | 2,5-Dichloranilin | 1,05 | 20,0 | 80/90 | 8/4 | 90,5 | 184 |
| 34 | 5-Amino-benzimidazolon | 1,05 | 10,0 | 80/90/100 | 4/4/5 | 95,9 | >320 |
| 35 | 2-Methoxy-5-methyl-4-chloranilin | 1,05 | 20,0 | 80 | 8 | 96,4 | 170 |
| 36 | 2,4-Dimethoxy-5-chlor-anilin | 1,05 | 20,0 | 80/90 | 5/8 | 89,3 | 186 |
| 37 | 2,5-Dimethoxy-4-chlor-anilin | 1,05 | 20,0 | 80/90 | 6/6 | 91,6 | 79 |
| 38 | N-Äthyl-anilin | 1,05 | 37,0 | 80 | 20 | 80,5 | 113 |

## Patentansprüche

1. Verfahren zur Herstellung von Monoarylthioharnstoff-Verbindungen durch Umsetzung von einem Salz eines Arylamins mit Ammoniumrhodanid oder einem Alkalirhodanid in wässrigem Medium, dadurch gekennzeichnet, dass man die Reaktion in einem Reaktionsgemisch ausführt, das ausgangs eine höhere Gewichtsmenge an dem Salz des Arylamins als an Wasser und die 1- bis 1,25-fach äquivalente Menge an dem Ammonium- oder Alkalirhodanid, bezogen auf das Arylamin, enthält sowie keine überschüssige Säure aufweist, und dass man die Reaktion bei einer Temperatur ausführt, die mindestens 5°C unterhalb des Siedepunktes des Reaktionsgemisches liegt.

2. Verfahren nach Anspruch 1, dadurch gekennzeichnet, dass man die Reaktion in Gegenwart von 5–10 Mol% (bezogen auf ein Mol Arylaminsalz) von Hydrogensulfit-Ionen vornimmt.

3. Verfahren nach Anspruch 1 oder 2, dadurch gekennzeichnet, dass das Ausgangs-Arylamin ein Anilin oder Naphthylamin ist, die durch schwach elektronegative, elektroneutrale und/oder elektropositive Substituenten substituiert sein können.

4. Verfahren nach Anspruch 3, dadurch gekennzeichnet, dass das Ausgangs-Arylamin ein Anilin oder Naphthylamin ist, die durch Substituenten substituiert sein können, die der Gruppe niederes Alkyl, Hydroxy, niederes Alkoxy, niederes Alkoxyalkyl, Phenoxy, durch niederes Alkyl, niederes Alkoxy und/oder Chlor substituiertes Phenoxy, Amino, durch niederes Alkyl und/oder Phenyl substituiertes Amino, Alkanoylamino, Benzoylamino, Alkylencarbonyloxyalkyl mit niederem Alkyl und Alkylen, niederes Alkylmercapto, Phenylthio, niederes Alkanoyloxy, Benzoyloxy, Halogen, niederes Alkanoyl, niederes Alkoxycarbonyl, Benzoyl, Carbamoyl, Sulfamoyl, durch niederes Alkyl und/oder Phenyl mono- oder disubstituiertes Carbamoyl und durch niederes Alkyl und/oder Phenyl mono- oder disubstituiertes Sulfamoyl angehören.

5. Verfahren nach Anspruch 1 oder 2, dadurch gekennzeichnet, dass man ein Arylamin der allgemeinen Formel (2)

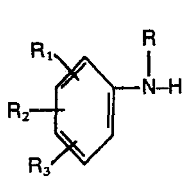

(2)

in welcher R für ein Wasserstoffatom oder eine niedere Alkylgruppe, $R_1$ für ein Wasserstoffatom oder ein Fluor-, Chlor- oder Bromatom oder eine niedere Alkylgruppe, eine niedere Alkoxygruppe, eine Hydroxy-, eine Amino-, Phenylamino-, Phenoxy-, niedere Monoalkylamino-, niedere Dialkylamino- oder für die Carbamoyl-Gruppe steht, $R_2$ ein Wasserstoffatom oder ein Chloratom oder eine niedere Alkylgruppe oder niedere Alkoxygruppe bedeutet und $R_3$ ein Wasserstoffatom oder eine niedere Alkylgruppe oder eine niedere Alkoxygruppe ist, wobei die Formelreste R, $R_1$, $R_2$ und $R_3$ gleich oder verschieden voneinander sein können, in einen Monophenyl-thioharnstoff der allgemeinen Formel (1)

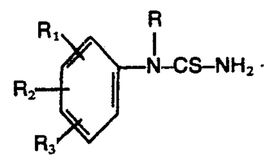

(1)

mit R, $R_1$, $R_2$ und $R_3$ der oben genannten Bedeutung überführt.

6. Verfahren nach einem der Ansprüche 1 bis 5, dadurch gekennzeichnet, dass man die Reaktion bei einer Temperatur von mindestens 50°C durchführt.

## Revendications

1. Procédé de préparation de monoaryl-thiourées par réaction d'un sel d'une arylamine avec le thiocyanate d'ammonium ou un thiocyanate de métal alcalin en milieu aqueux, procédé caractérisé en ce qu'on effectue la réaction dans un mélange réactionnel qui contient au départ, en poids, davantage du sel de l'arylamine que d'eau et de 1 à 1,25 fois la quantité équivalente du thiocyanate d'ammonium ou de métal alcalin par rapport à l'arylamine et, qui ne contient pas d'acide en excès, à une température inférieure d'au moins 5°C au point d'ébullition du mélange réactionnel.

2. Procédé selon la revendication 1, caractérisé en ce qu'on effectue la réaction en présence de 5 à 10% en moles (par rapport à 1 mole du sel d'arylamine) d'ions hydrogéno-sulfite.

3. Procédé selon l'une des revendications 1 et 2, caractérisé en ce que l'arylamine de départ est une aniline ou une naphtylamine qui peuvent porter des substituants légèrement électronégatifs, électriquement neutres et/ou électropositifs.

4. Procédé selon la revendication 3, caractérisé en ce que l'arylamine de départ est une aniline ou une naphtylamine qui peuvent porter des substituants pris parmi des radicaux alkyles inférieurs, le groupe hydroxy, des radicaux alcoxy inférieurs, des radicaux alcoxy-alkyles inférieurs, le radical phénoxy, les radicaux phénoxy porteurs d'un alkyle inférieur, d'un alcoxy inférieur et/ou d'un atome de chlore, le groupe amino, les groupes amino porteurs d'un alkyle inférieur et/ou d'un phényle, des radicaux alcanoylamino et benzoylamino, les radicaux alkylène-carbonyloxy-alkyles à alkyle et alkylène inférieurs, les radicaux alkylthio inférieurs, le radical phénylthio, les radicaux alcanoyloxy inférieurs, le radical benzoyloxy, les halogènes, les radicaux alcanoyles inférieurs, les radicaux alcoxycarbonyles inférieurs, les radicaux benzoyle, carbamoyle et sulfamoyle, les radicaux carbamoyles porteurs d'un ou deux substituants pris parmi les alkyles inférieurs et le radical phényle, et les radicaux sulfamoyles porteurs d'un ou deux substituants pris parmi les alkyles inférieurs et le radical phényle.

5. Procédé selon l'une des revendications 1 et 2, caractérisé en ce qu'on transforme une arylamine répondant à la formule générale 2:

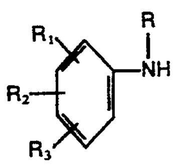
(2)

dans laquelle:
R représente un atome d'hydrogène ou un radical alkyle inférieur,
$R_1$ représente un atome d'hydrogène, de fluor, de chlore ou de brome, un radical alkyle inférieur, un radical alcoxy inférieur, un radical hydroxy, amino, phénylamino ou phénoxy, un radical monoalkylamino inférieur, un radical dialkylamino inférieur ou un radical carbamoyle,
$R_2$ représente un atome d'hydrogène ou de chlore, un radical alkyle inférieur ou un radical alcoxy inférieur, et
$R_3$ représente un atome d'hydrogène, un radical alkyle inférieur ou un radical alcoxy inférieur,
les radicaux R, $R_1$, $R_2$ et $R_3$ pouvant être identiques ou différents les uns des autres,
en une monophényle-thio-urée répondant à la formule générale 1:

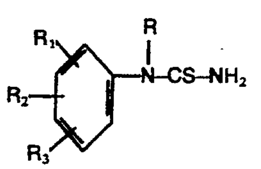
(1)

dans laquelle R, $R_1$, $R_2$ et $R_3$ ont les significations précédemment données.

6. Procédé selon l'une quelconque des revendications 1 à 5, caractérisé en ce qu'on effectue la réaction à une température d'au moins 50 °C.

**Patent Claims**

1. Process for the preparation of monoaryl thiourea compounds by reacting a salt of an arylamine with ammonium rhodanide or an alkali metal rhodanide in an aqueous medium, characterized by that the reaction is carried out in a reaction mixture which at the start contains a higher amount by weight of the salt of the arylamine than of water and from 1 to 1.25 times the equivalent amount of the ammonium rhodanide or alkali metal rhodanide calculated on the arylamine, as well as does not have excess acid, and that the reaction is carried out at a temperature which is at least 5 °C below the boiling point of the reaction mixture.

2. Process according to claim 1, characterized by that the reaction is carried out in presence of from 5 to 10 mol% (calculated on one mol of the salt of the arylamine) of hydrogenosulfite ions.

3. Process according to claim 1 or 2, characterized by that the starting arylamine is an aniline or a naphthylamine, which may be substituted by slightly electronegative, electroneutral and/or electropositive substituents.

4. Process according to claim 3, characterized by that the starting arylamine is an aniline or a naphthylamine, which may be substituted by substituents belonging to the group of lower alkyl, hydroxy, lower alkoxy, lower alkoxyalkyl, phenoxy, phenoxy substituted by lower alkyl, lower alkoxy and/or chlorine, amino, amino substituted by lower alkyl and/or phenyl, alkanoylamino, benzoylamino, alkylene-carbonyloxyalkyl with lower alkyl and alkylene, lower alkylmercapto, phenylthio, lower alkanoyloxy, benzoyloxy, halogen, lower alkanoyl, lower alkoxycarbonyl, benzoyl, carbamoyl, sulfamoyl, carbamoyl mono- or disubstituted by lower alkyl and/or phenyl, and sulfamoyl mono- or disubstituted by lower alkyl and/or phenyl.

5. Process according to claim 1 or 2, characterized by that an arylamine of the general formula (2)

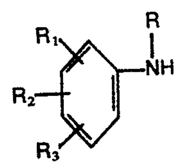
(2)

in which R is an hydrogen atom or a lower alkyl group, $R_1$ is a hydrogen atom or a fluorine-, chlorine- or bromine atom or a lower alkyl group, a lower alkoxy group, a hydroxy-, an amino-, phenylamino-, phenoxy-, lower monalkylamino-, lower dialkylamino- or the carbamoyl group, $R_2$ is a hydrogen atom or a chlorine atom or a lower alkyl group or lower alkoxy group, and $R_3$ is a hydrogen atom or a lower alkyl group or a lower alkoxy group, and the formula radicals R, $R_1$, $R_2$ and $R_3$ may be identical or different from one another, is converted into a monophenyl thiourea of the general formula (1)

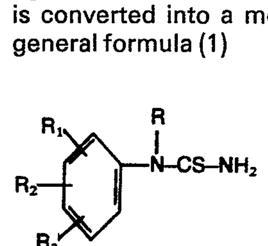
(1)

in which R, $R_1$, $R_2$ and $R_3$ have the above meaning.

6. Proces according to any one of the claims 1 to 5 characterized by that the reaction is carried out at a temperature of at least 50 °C.